# EUROPEAN PATENT APPLICATION

(11) **EP 2 221 532 A2**
(43) Date of publication of application: **25.08.2010**
(21) Application number: 09171229.9
(22) Date of filing: 24.09.2009
(51) Int. Cl.: F21V 21/26, F21S 6/00, F21Y 101/02, F21W 131/30

(54) **Lamp assembly and heat dissipating lampshade**

(30) Priority: 19.02.2009 TW 98202382 U
(71) Applicant: Advanced Connectek Inc., Taipei Hsien Hsin-Tien (TW)
(72) Inventor: Chien, Wen-Hsiang, Hsin-Tien (TW); Chen, Chih-Hung, Hsin-Tien (TW)
(74) Representative: Ihle, Kornelia

(57) **Abstract**

A lamp assembly includes a bottom seat and a lampshade. The lampshade includes a lamp cover holding a light emitting element therein, and a connecting arm having a first end connected to the lamp cover and a second end connected pivotally to the bottom seat at a pivot point thereof. The lamp cover and the connecting arm are integrally formed with each other as one-piece unit.

## Description

This application claims the benefits of the Taiwan Patent Application Serial NO. 098202382, filed on February 19, 2009, the subject matter of which is incorporated herein by reference.

### BACKGROUND OF THE INVENTION

### 1. Field of the Invention

The present invention relates to a lamp assembly, more particularly to a lamp assembly including a lampshade capable of dissipating heat effectively therefrom.

### 2. Description of the Prior Art

A lamp assembly plays an important role in our daily life. For instance, desktop lamp or table lamp we usually used at home or office generally includes a light emitting element for emitting light. In the past, an incandescent light bulb or tube is usually used and consumes relatively large energy. In order to promote energy saving and carbon emission reduction measures, the incandescent bulbs or tubes are gradually replaced with LEDs (Light Emitting Diode), since the latter consume lesser energy, radiate lower heat and provide longer service life when compare to the former ones.

Generally speaking, in order for an LED (Light Emitting Diode) to dissipate heat effectively, a heat sink is usually mounted at a rear side thereof. The heat sink is mostly made from aluminum, includes a plurality of radiating fins of complicated structure. Since the electronic products are in the trend of slim type day by day, the complicated structure of the radiating fins and its thickness thereof are not welcomed by the manufacturers. However while designing the product to be slim and thin, the heat dissipation ability of the product should not be neglected also.

Therefore, it is the object of the present invention to provide a light assembly having a heat dissipating lampshade that is free from the drawbacks encountered during use of the prior art light assembly.

### SUMMARY OF THE INVENTION

One aspect of the present invention is to provide a lamp assembly having a heat dissipating lampshade of slim type to enable effective heat dissipation ability, more particularly an LED is implemented as the light emitting element.

The lamp assembly of the present invention includes a bottom seat and a lampshade disposed above and connected pivotally to the bottom seat.

The lampshade preferably includes a lamp cover holding a light emitting element therein, and a connecting arm having a first end connected to the lamp cover and a second end connected pivotally to the bottom seat at a pivot point thereof, wherein, the lamp cover and the connecting arm are integrally formed with each other as one-piece unit. The lampshade further includes a curved portion interconnecting the first and second ends of the connecting arm. An LED (Light Emitting Diode) serves as the light emitting element.

Since the lampshade employed in the lamp assembly of the present invention is in the form of one-piece unit, it is slim in thickness, and has aesthetic appearance and provides effective heat dissipation ability, thereby prolonging the service life of the lamp assembly of the present invention.

### BRIEF DESCRIPTION OF THE DRAWINGS

Other features and advantages of this invention will become more apparent in the following detailed description of the preferred embodiment of this invention, with reference to the accompanying drawings, in which:

Figure 1 is a perspective view of a lamp assembly of the present invention;

Figure 2 is an enlarge and fragmentary view of a lampshade employed in the lamp assembly shown in Figure 1; and

Figure 3 is a lateral side view of the lampshade shown in Figure 2, illustrating how it dissipates heat therefrom.

### DETAILED DESCRIPTION OF THE PREFERRED EMBODIMENT

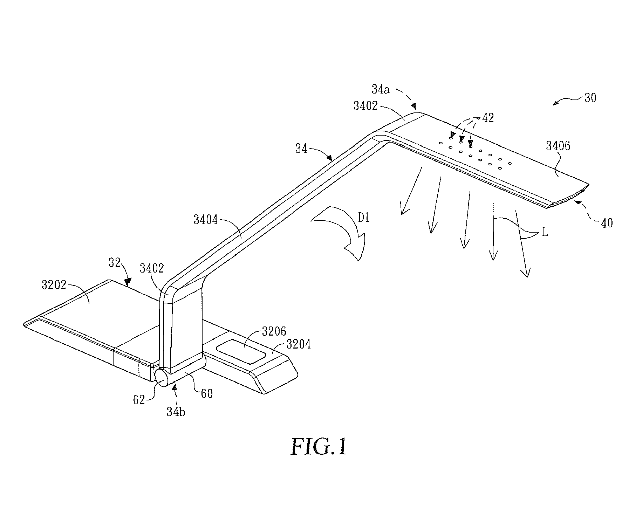

Figure 1 is a perspective view of a lamp assembly 30 of the present invention, and includes a bottom seat 32 and a heat dissipating lampshade 34.

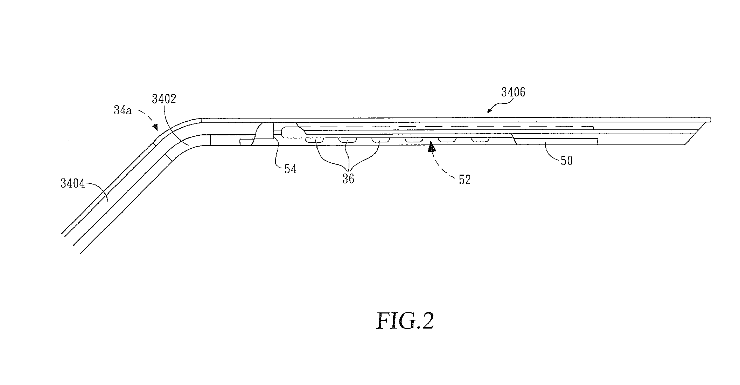

The lampshade 34 includes a lamp cover 3406 and a connecting arm 3404 integrally formed with the lamp cover 3406. The lamp cover 3406 is provided with a light emitting element 36, which emits light beams L therefrom. Preferably, a set of LEDs (Light Emitting Diode) serves as the light emitting element 36 (see Fig. 2). The connecting arm 3404 has a first end 34a connected securely to the lamp cover 3406 and a second end 34b connected pivotally to the bottom seat 32 at a pivot point thereof. Preferably, the lamp cover 3406 and the connecting arm 3404 are integrally formed as one-piece unit. Under this condition, the lampshade 34 is turnable toward the user along the direction D1 so as to adjust the angle of the light beam L relative to the bottom seat 32.

The lamp cover 3406 is formed with a plurality of ventilation holes 42 located above the first end 34a of the connecting arm 3404 and the emitted light beam L, and the heat generated from the light emitting diodes can pass through the ventilation holes 42 due to evaporation phenomenon thereof. The ventilation holes 42 at the upper portion of the lamp cover 3406 are arranged to provide aesthetic beauty also.

The bottom seat 32 includes a seat body 3202 and an extension portion 3204 projecting outward from a periphery of the seat body 3202 along a direction D1 of the curved portion 3402. Thus, when the lampshade 34 is bent toward the user side in order to adjust the angle of the light beam L, the extension portion 3204 provides a stable support against the turning force, thereby preventing overturn of the lamp assembly 30 of the present invention. The pivot point of the connecting arm 3404 is located to adjacent to the periphery of the seat body 3202

The bottom seat 32 further has a control panel 3206 disposed on an upper surface of the extension portion 3204. The control panel 3206 may include switch buttons (not visible), light adjustment knob for controlling the electronic components and the like and is designed ergonomically so as to extend toward the user to facilitate manipulating of the same.

In this embodiment, the second end 34b of the connecting arm 3404 is connected pivotally to the seat body 3202 via a pivot unit. The pivot unit includes a mounting shaft 62 fixed securely to the seat body 3202 of the bottom seat 32 and a tubular sleeve 60 connected securely to the second end 34b of the connecting arm 3404 and sleeved frictionally around the mounting shaft 62. Under this condition, the lampshade 34 is turnable relative to and position above the bottom seat 32

The lampshade 34 further includes two curved portion 3402 interconnecting the first and second ends 34a, 34b of the connecting arm 3404. The upper curved portion 3402 is located at a position adjoining the connecting arm 3404 and the lamp cover 3406 and is bent in the direction D1 towards the bottom seat 32 when compared to the lower curved portion 3402 located adjacent to the bottom seat 32. Thus, the lower curved portion 3402 is generally intended for stable gravity of the connecting arm 3404 while the upper curved portion 3402 is intended for adjusting the angle of the light beam L in the direction D1.

To be more specific, the lampshade 34 is movable relative to the bottom seat 32 between a distal position (the farthest position) and a proximate position (the nearest position). Note that when the lampshade 34 is rotated to the distal position, the gravity center of the lampshade 34 is perpendicular to a horizontal line passing through the bottom seat 32 so that when the lampshade 34 rotated toward the bottom seat 32 from the distal position, it is stable on the bottom seat 32. Thus, the lamp assembly 30 of the present invention is prevented from collapsing during use (i.e. when adjusting the lampshade 34 relative to the bottom seat 32).

Considering the manufacturing expense and the aesthetic appearance, the lampshade 34 employed in the lamp assembly 30 of the present invention is made from aluminum. Preferably, the lamp cover 3406 can be a hollow body that is made from bending an aluminum sheet and that has a distal portion formed with an opening 40 to facilitate heat dissipation.

Alternately, the lamp cover 3406 can be a hollow body that is made from compressing an aluminum sheet and that has a distal portion formed with an opening 40 for heat dissipation. In addition, the lamp cover 3406 and the connecting arm 3404 constituting the lampshade 34 can be formed by laser cutting process.

Figure 2 shows an enlarge and fragmentary view of the lampshade 34 employed in the lamp assembly 30 shown in Figure 1. As illustrated, the lamp cover 3406 is formed with a bulb-receiving channel 52, within which the light emitting element 36 is seated. The lampshade 34 further includes a transparent plate 50 mounted to the lamp cover 3406 for shielding the bulb-receiving channel 52.

The lampshade 34 further includes a coupler socket 54 installed within the bulb-receiving channel 52 of the lamp cover 3405 and is adapted to be connected to a power source. The light emitting element 36 is inserted into the coupler socket 52 of the lamp cover 3405 as a module.

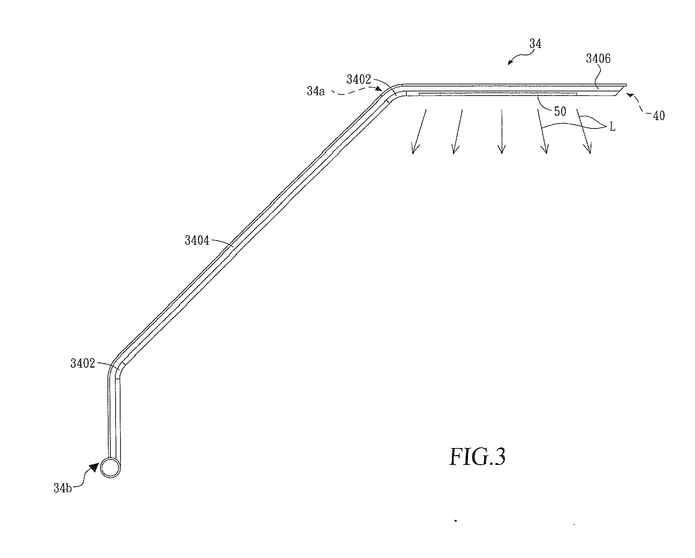

Figure 3 is a lateral side view of the lampshade 34 employed in the lamp assembly 30 shown in Figure 1, illustrating how it dissipates heat therefrom. The lampshade 34 is a one-piece unit and includes the lamp cover 3406 connected to the first end 34a of the connecting arm 3404. The light emitting element 36 (preferably a set of LEDs) is mounted in the lamp cover 3406. The second end 34b of the connecting arm 3404 is connected pivotally at a pivot point thereof in such a manner to permit pivotal movement of the lamp cover 3406. In the drawing, the connecting arm 3404 should not have two curved portions 3402 when the same is not mounted securely on table like bottom seat 32.

In other words, the lower curved portion 34b of the connecting arm 3404 can be connected securely to a study table, the head of the bedding, a settee in the drawing room or any other suitable places. As shown in Figure 1, unlike to the conventional lampshade of two-piece formation, the lampshade 34 employed in the present invention is formed as a one-piece metal unit to provide aesthetic appearance and a larger heat dissipating effect.

In order to economize the material cost and to enhance the heat dissipation effect, the lampshade 34 employed in the lamp assembly 30 of the present invention is made from aluminum. Preferably, the lamp cover 3406 can be a hollow body that is made from bending or compressing an aluminum sheet and that has a distal portion formed with an opening 40 to facilitate heat dissipation. Because, the lampshade 34 has curved portions at the intermediate section, a specially designed auxiliary tool will be needed to form consistency of the structure of the lampshade 34.

In addition, the ventilation holes 42 in the lamp cover 3406 are located above the emitted light beam L and the heat generated from the light emitting element 36 can pass through the ventilation holes 42 due to evaporation phenomenon thereof.

In addition, the transparent plate 50 is mounted to the lamp cover 3406 for shielding the bulb-receiving channel 52, where the light emitting element 36 is mounted. Thus, the light emitting element 36 is prevented from being contaminated.

Since the lampshade 34 of the lamp assembly 30 of the present invention is an integrally formed one-piece unit, it possesses a relatively thinness and provides effective heat dissipation ability in addition to its external appearance, thereby prolonging the service life of the LEDs.

While the invention has been described in connection with what is considered the most practical and preferred embodiments, it is understood that this invention is not limited to the disclosed embodiments but is intended to cover various arrangements included within the spirit and scope of the broadest interpretation so as to encompass all such modifications and equivalent arrangements.

## Claims

1. A lamp assembly comprising:
a bottom seat; and
a lampshade including
a lamp cover holding a light emitting element therein, and
a connecting arm having a first end connected to said lamp cover and a second end connected pivotally to said bottom seat at a pivot point thereof;
wherein, said lamp cover and said connecting arm are integrally formed with each other as one-piece unit.

2. The lamp assembly according to claim 1, wherein said lampshade further includes a first curved portion interconnecting said first and second ends of said connecting arm.

3. The lamp assembly according to claim 2, wherein said bottom seat includes a seat body and an extension portion projecting outward from a periphery of said seat body along a direction of said curved portion, said pivot point of said connecting arm being located adjacent to the periphery of said seat body.

4. The lamp assembly according to claim 3, wherein said bottom seat further has a control panel disposed on an upper surface of said extension portion.

5. The lamp assembly according to claim 1, wherein said lamp cover is a hollow body that is made from bending an aluminum sheet and that has a distal portion formed with an opening for heat dissipation.

6. The lamp assembly according to claim 1, wherein said lamp cover is a hollow body that is made from compressing an aluminum sheet and that has a distal portion formed with an opening for heat dissipation.

7. The lamp assembly according to claim 1, wherein said lamp cover is formed with a plurality of ventilation holes located above said light emitting element.

8. The lamp assembly according to claim 1, wherein said light emitting element is an LED (Light Emitting Diode).

9. The lamp assembly according to claim 1, wherein said lamp cover is formed with a bulb-receiving channel, within which said light emitting element is seated, said lampshade further including a transparent plate mounted to said lamp cover for shielding said bulb-receiving channel.

10. The lamp assembly according to claim 9, wherein said lampshade further includes a coupler socket installed within said bulb-receiving channel and electrically connected to a power source, said light emitting element being inserted into said coupler socket.

11. The lamp assembly according to claim 1, wherein said second end of said connecting arm is connected pivotally to said bottom seat via a pivot unit, said pivot unit including a mounting shaft fixed securely to said bottom seat and a sleeve connected securely to said second end of said connecting arm and sleeved rotatably around said mounting shaft.

12. The lamp assembly according to claim 2, wherein said lampshade further includes a second curved portion interconnecting said first and second ends of said connecting arm.

13. The lamp assembly according to claim 1, wherein said lampshade is movable relative to said bottom seat between a distal position and a proximate position, the gravity center of said lampshade at said distal position being perpendicular to a horizontal line passing through said bottom seat.

14. A lampshade for use in a lamp assembly, comprising:
a lamp cover holding a light emitting element therein; and
a connecting arm having a first end connected to said lamp cover and a second end connected pivotally at a pivot point thereof in such a manner to permit pivotal movement of said lamp cover;
wherein, said lamp cover and said connecting arm are integrally formed with each other as one-piece unit.

15. The lampshade according to claim 14, further comprising a curved portion interconnecting said first and second ends of said connecting arm.

16. The lampshade according to claim 14, wherein the lamp assembly includes a bottom seat disposed below the lampshade, said second end of said connecting arm being pivotally connected to the bottom seat.

17. The lampshade according to claim 16, wherein the bottom seat includes a seat body and an extension portion projecting outward from a periphery of said seat body along a direction of said curved portion, said pivot point of said connecting arm being located to adjacent to the periphery of said seat body.

18. The lampshade according to claim 17, wherein the bottom seat further has a control panel disposed on an upper surface of said extension portion.

19. The lampshade according to claim 14, wherein said lamp cover is a hollow body that is made from bending an aluminum sheet and that has a distal portion formed with an opening for heat dissipation.

20. The lampshade according to claim 14, wherein said lamp cover is a hollow body that is made from compressing an aluminum sheet and that has a distal portion formed with an opening for heat dissipation.

21. The lampshade according to claim 14, wherein said lamp cover is formed with a plurality of ventilation holes located above said light emitting element.

22. The lampshade according to claim 14, wherein said light emitting element is an LED (Light Emitting Diode).

23. The lampshade according to claim 14, wherein said lamp cover is formed with a bulb-receiving channel, within which said light emitting element is seated, the lampshade further comprising a transparent plate mounted to said lamp cover for shielding said bulb-receiving channel.

24. The lampshade according to claim 23, further comprising a coupler socket installed within said bulb-receiving channel and electrically connected to a power source, said light emitting element being inserted into said coupler socket.

25. The lampshade according to claim 16, wherein said second end of said connecting arm is connected pivotally to the bottom seat via a pivot unit, said pivot unit including a mounting shaft fixed securely to the bottom seat and a sleeve connected securely to said second end of said connecting arm and sleeved rotatably around said mounting shaft.

26. The lampshade according to claim 15, further comprising a second curved portion interconnecting said first and second ends of said connecting arm.

27. The lampshade according to claim 16, wherein said connecting arm is movable relative to said bottom seat between a distal position and a proximate position, the gravity center of said connecting arm and said lamp cover at said distal position being perpendicular to a horizontal line passing through said bottom seat.
